# EUROPEAN PATENT APPLICATION

(11) **EP 4 545 522 A1**
(43) Date of publication of application: **30.04.2025**
(21) Application number: 23826863.5
(22) Date of filing: 23.05.2023
(51) Int. Cl.: C07D 405/14, C08F 26/00, C08L 39/00, C07D 307/14

(54) **COMPOUND, COMPOSITION, CURED PRODUCT, AND COMPOUND MANUFACTURING METHOD**

(30) Priority: 21.06.2022 JP 2022099809
(71) Applicant: MITSUBISHI GAS CHEMICAL COMPANY, INC., Chiyoda-ku Tokyo 100-8324 (JP)
(72) Inventor: FUJII, Shiki, Tokyo 125-8601 (JP); OHNO, Daisuke, Tokyo 100-8324 (JP); KITAMURA, Mitsuharu, Tokyo 125-8601 (JP)
(74) Representative: Müller-Boré & Partner Patentanwälte PartG mbB
(86) International application number: PCT/JP2023/019051
(87) International publication number: WO 2023/248675

(57) **Abstract**

To provide a compound represented by Formula (1): wherein the dotted line portions each independently represent *-CH-CH₂- or *-C=CH-, and * is bonded to the side near the oxygen atom contained in the ring.

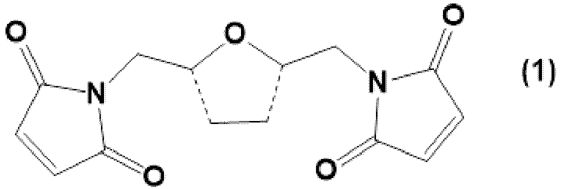

## Description

### [Technical Field]

The present invention relates to a compound, a composition, a cured product, and a method for producing the compound.

### [Background Art]

Bismaleimide resin is generally known as a highly heat resistant thermosetting resin and is widely used in the fields of molding materials, composite materials, electrical and electronic components, and the like. In the field of recent electrical and electronic components, the development of microelectronics has been remarkable. Especially for large computers, high-speed signal transmission is essential due to the introduction of multilayer circuit boards and the like, but if the dielectric constant of a substrate material is large, the delay of signal transmission will occur and become an obstacle to achieving higher speeds. Bismaleimide is used as an interlayer insulating film in multilayer wiring structures. For these reasons, the need for bismaleimide that maintains heat resistance and has a low dielectric constant has gained prominent attention.

In Patent Literature 1, the bismaleimide resin shown below is disclosed as bismaleimide resin suitable for electronic material applications. In the following structural formula, X and Y are structures containing a predetermined aromatic ring, and a and b are integers of 0 to 100, of which at least one is not 0.

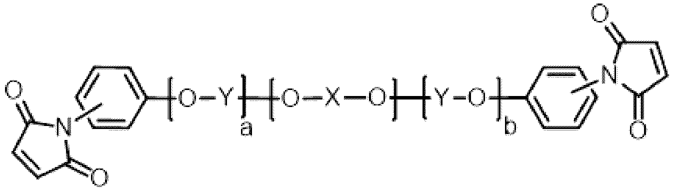

### [Citation List]

### [Patent Literature]

[Patent Literature 1]
Japanese Patent Laid-Open No. 2010-018791

### [Summary of Invention]

### [Technical Problem]

Although the above bismaleimide resin is an excellent material, there is a need to further improve the functionality of maleimide compounds along with the recent demand for higher performance in the field of electronic materials. In particular, there is a need for maleimide compounds having high heat resistance and a low coefficient of thermal expansion (CTE).

The present invention aims to solve such problems, and to provide a maleimide compound having a low coefficient of thermal expansion (CTE) while maintaining excellent heat resistance, and a composition, a cured product, and a method for producing the compound.

### [Solution to Problem]

The present inventors conducted research to address the above-mentioned problems, and as a result, the problems described above are solved by the following means.
<1> A compound represented by Formula (1): 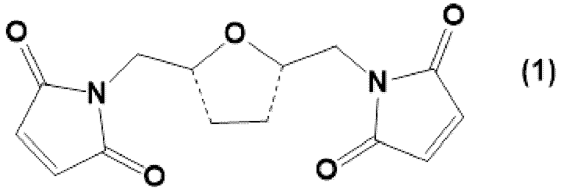 wherein the dotted line portions each independently represent *-CH-CH₂- or *-C=CH-, and * is bonded to the side near the oxygen atom contained in the ring.
<2> The compound according to <1>, wherein the compound represented by Formula (1) is a compound represented by Formula (2): 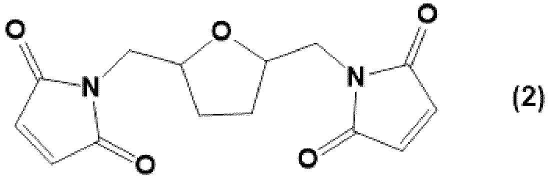
<3> A compound represented by Formula (4): 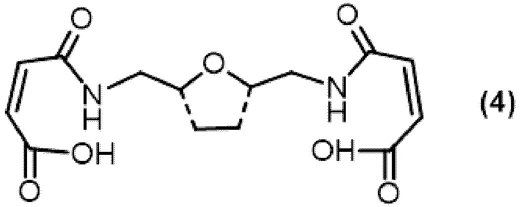 wherein the dotted line portions each independently represent *-CH-CH₂- or *-C=CH-, and * is bonded to the side near the oxygen atom contained in the ring.
<4> A composition comprising the compound according to <1> or <2>.
<5> A cured product formed from the composition according to <4>.
<6> A method for producing the compound according to <1> or <2>, the method comprising using an intermediate represented by Formula (4): 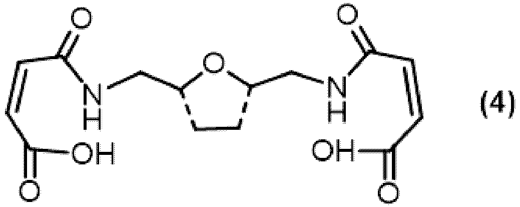 wherein the dotted line portions each independently represent *-CH-CH₂- or *-C=CH-, and * is bonded to the side near the oxygen atom contained in the ring.

### [Advantageous Effects of Invention]

According to the present invention, a compound having a low coefficient of thermal expansion (CTE) while maintaining excellent heat resistance, and a composition, a cured product, and a method for producing the compound have been provided.

### [Brief Description of Drawings]

[Figure 1] Figure 1 shows NMR spectra of an intermediate synthesized in Example 1.
[Figure 2] Figure 2 shows NMR spectra of a compound represented by Formula (2) in Example 1.

### [Description of Embodiment]

Hereinafter, an embodiment for implementing the present invention (hereinafter simply referred to as "the present embodiment") will be described in detail. Also, the following embodiment is an example for describing the present invention, and the present invention is not limited to the present embodiment only.

Still, in the present specification, "to" is used in the sense that it includes the numerical numbers described before and after it as lower and upper limits.

In the present specification, various physical properties and characteristic values are those obtained at 23°C, unless otherwise stated.

In the present specification, relative permittivity is a ratio of a dielectric constant of a substance to the dielectric constant of vacuum. In the present specification, the relative permittivity is sometimes simply referred to as " dielectric constant ".

When the standards referred to herein differ in measurement methods and the like depending on the year, they are based on the standards as of January 1, 2022, unless otherwise stated.

### <A compound represented by Formula (1)>

A compound of the present embodiment is represented by Formula (1). Such a compound, when made into a cured product, can effectively achieve a low coefficient of thermal expansion (CTE) while maintaining excellent heat resistance. 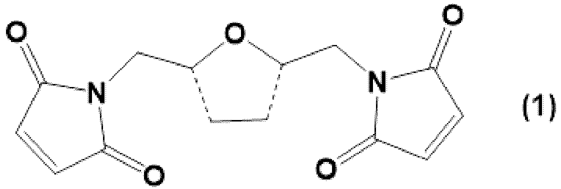 wherein the dotted line portions each independently represent *-CH-CH₂- or *-C=CH-, and * is bonded to the side near the oxygen atom contained in the ring.

In the compound represented by Formula (1), the dotted line portions each independently represent *-CH-CH₂- or *-C=CH-.

In the present embodiment, the compound represented by Formula (1) is represented by the following Formula (2) or Formula (3), preferably represented by the Formula (2).

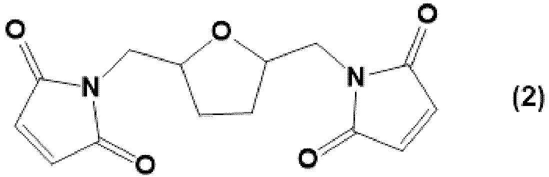

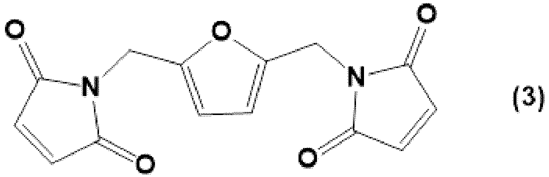

### <A method for producing the compound represented by Formula (1)>

The compound represented by Formula (1) can be produced, for example, by reacting 2,5-bis(aminomethyl)tetrahydrofuran (hereinafter sometimes referred to as aminomethylfuran A) and/or 2,5-bis(aminomethyl)furan (hereinafter sometimes referred to as aminomethylfuran B) and maleic anhydride under a solvent. In this instance, a catalyst may be used.

Examples of the catalyst include toluene sulfonic acid hydrate, phosphoric acid, and sulfuric acid, preferably the toluene sulfonic acid hydrate.

Examples of the solvent include an aromatic hydrocarbon solvent, an amide solvent, an ether solvent, an alcohol solvent, and a halogen solvent, preferably the aromatic hydrocarbon solvent and the amide solvent, and more preferably N-methyl-2-pyrrolidone and toluene. These organic solvents may be used alone or in a mixture of two or more.

Hereinafter, an example of a specific production method of the compound represented by Formula (1) is described.

The aminomethylfuran A and/or B and the maleic anhydride are each dissolved in a solvent, and a solution of the aminomethylfuran A and/or B is added (e.g., dropwise) to a maleic anhydride solution to produce an intermediate represented by the following Formula (4): 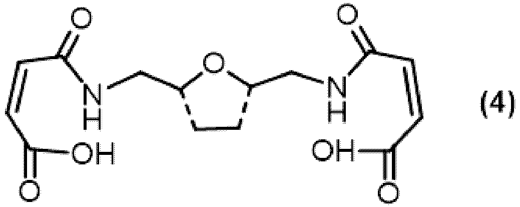 wherein the dotted line portions each independently represent *-CH-CH₂- or *-C=CH-, and * is bonded to the side near the oxygen atom contained in the ring.

An upper limit of a reaction temperature when the above described intermediate of Formula (4) is produced by reacting of the aminomethylfuran A or B with the maleic anhydride is preferably 60°C or less, and more preferably 50°C or less. In addition, a lower limit of the reaction temperature is preferably 0°C or more, and more preferably 10°C or more.

Furthermore, reaction time is preferably 0.5 hours or more and 100 hours or less, and more preferably 5 hours or more and 48 hours or less.

The resulting intermediate is then heated under a catalyst for certain time to produce the compound represented by Formula (1).

An upper limit of a reaction temperature when the compound represented by Formula (1) is produced from the above intermediate of Formula (4) is preferably 250°C or less, and more preferably 200°C or less. In addition, a lower limit of the reaction temperature is preferably 25°C or more, and more preferably 100°C or more.

Furthermore, an upper limit of reaction time is preferably 24 hours or less, and more preferably 15 hours or less. In addition, a lower limit of the reaction time is preferably 1 hour or more.

For the reaction liquid obtained above, purification and the like may be performed after completion of the reaction to purify the compound represented by Formula (1).

The aminomethylfuran A and/or B used may be those obtained by the synthetic method described in paragraphs 0011 to 0016 of International Publication No. WO 2019/073987, the contents of which are incorporated herein. Commercially available products may also be used.

### <A composition including the compound represented by Formula (1)>

A composition of the present embodiment includes the compound represented by Formula (1).

An upper limit of a content of the compound represented by Formula (1) in the composition is preferably 100 parts by mass or less with respect to 100 parts by mass of a resin solid content. In addition, a lower limit of the content is preferably 0.1 parts by mass or more.

The composition of the present embodiment may include only one compound represented by Formula (1), or may include two or more compounds. When two or more compounds are included, the total content is preferably within the range described above.

The resin solid content refers to the component excluding a filler and a solvent, and includes the compound represented by Formula (1) and other resin additive components (additives and the like, such as a flame retardant).

The composition of the present embodiment may include a thermosetting compound other than the compound represented by Formula (1). Examples of the thermosetting compound include an epoxy resin, an oxetane resin, a compound having a polymerizable unsaturated group, a cyanate ester compound, a benzoxazine resin, and a phenolic compound.

As specific thermosetting compounds, reference may be made to the compounds described in paragraphs 0034 to 0039 of Japanese Patent Laid-Open No. 2010-018791 and paragraphs 0047 to 0056 of International Publication No. WO 2021/172317, the contents of which are incorporated herein.

The composition of the present embodiment may include other components other than the thermosetting compound. Examples of other components include a filler, a solvent, a thermoplastic resin (including a thermoplastic elastomer), and resin additives such as a photo and/or thermal polymerization initiator, a photo sensitizing agent, a curing agent, a coupling agent, a coloring pigment, a defoaming agent, a surface modifier, a flame retardant, a dispersant, a curing accelerator, an ultraviolet absorbent, an antioxidant, and a flow control agent.

As specific other components, reference may be made to the compounds described in paragraphs 0040 to 0051 of Japanese Patent Laid-Open No. 2010-018791 and paragraphs 0061 to 0076 of International Publication No. WO 2021/172317, the contents of which are incorporated herein.

Examples of the solvent include ketones, Cellosolves, esters, amides, and aromatic hydrocarbons. As specific solvents, reference may be made to those described in paragraph 0077 of International Publication No. WO 2021/172317, the contents of which are incorporated herein.

### <A cured product formed from the composition including the compound represented by Formula (1)>

A cured product of the present embodiment is obtained by curing the composition described above in accordance with a known method, for example, a curing method by heat, an ultraviolet ray, or an electron beam. When curing by the heat, after filling the composition within a temperature range of a molten state in a die or the like, the temperature is raised above a predetermined polymerization temperature to promote a cross-linking reaction, to obtain the cured product. When curing by using the ultraviolet ray, a low-pressure mercury lamp, a medium-pressure mercury lamp, a high-pressure mercury lamp, an ultra-high-pressure mercury lamp, a xenon lamp or a metal halide lamp, etc. can be used as a light source for the ultraviolet ray.

A curing time of the composition when obtaining the cured product of the present embodiment is preferably 0.1 hours or more and 5 hours or less. Also, a curing temperature is preferably 100°C or more and 300°C or less.

The cured product of the present embodiment has a glass transition temperature (Tg) according to DMA (Dynamic Mechanical Measurement) of preferably 150°C or more, and more preferably 250°C or more. Such a high glass transition temperature is mainly achieved by using the compound represented by Formula (1). An upper limit of the glass transition temperature is, for example, 400°C or less in practice.

The glass transition temperature (Tg) is measured by the method described in Examples described later.

It is preferable that the cured product of the present embodiment has a low coefficient of thermal expansion (CTE). Specifically, the coefficient of linear thermal expansion in the direction perpendicular to the plane measured by the TMA method specified in JIS C 6481 5.19 when formed into a cured plate of 1 mm thickness is preferably 100 ppm/°C or less, and more preferably 80 ppm/°C or less. A lower limit is ideally 0 ppm/°C, but in practice 50 ppm/°C or more.

The measurement of CTE is performed by the method described in Examples described later.

The compound represented by Formula (1) thus obtained, the composition described above, or the cured product described above is useful for various applications such as an insulating material for printed wiring boards, a resin for resists, a semiconductor packaging material, a resin for semiconductor encapsulation, an adhesive for printed wiring boards, a build-up laminate material, a resin for fiber reinforced plastics, a resin for encapsulation of liquid crystal display panels, a resin for color filters of liquid crystals, a paint, a variety of coating agents, and an adhesive.

### [Examples]

Hereinafter, the present invention will be described more specifically with Examples. Materials, amounts used, proportions, processing details, processing procedures, etc. shown in the following Examples may be modified as appropriate, as long as they do not depart from the intent of the present invention. Therefore, the scope of the present invention is not limited to the specific examples shown below.

In case that measuring instruments and the like used in Examples are not available due to discontinuation or other reasons, the measurement may be performed using other instruments having equivalent performance.

### <Synthesis example of H-AMF>

Tetrahydrofuran (H-AMF, molecular weight 130.19) used in the present Examples was synthesized in accordance with paragraph 0033 of International Publication No. WO 2019/073987.

### <Example 1>

In a 200 mL flask, 45.19 g of maleic anhydride (produced by FUJIFILM Wako Pure Chemical Corporation, molecular weight 130.19), 80 mL of N-methyl-2-pyrrolidone (NMP, produced by KANTO CHEMICAL CO., INC.), and 80 mL of toluene (produced by KANTO CHEMICAL CO., INC.) were added and stirred. Then, 20.00 g of H-AMF synthesized above was dissolved in 40 mL of NMP and dropped into the above flask for 70 minutes and stirred overnight at room temperature.

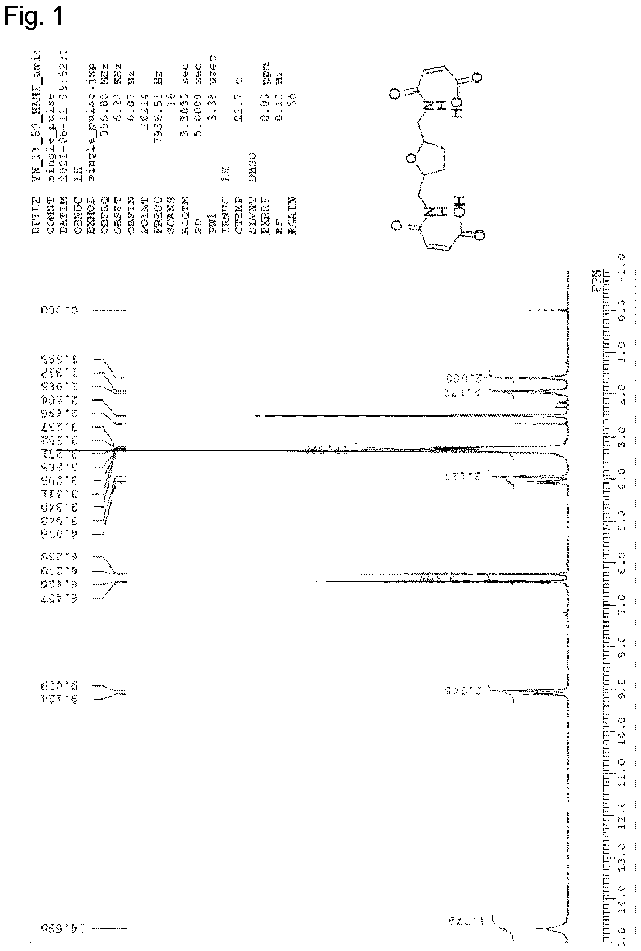

After overnight, sampling was performed and NMR of the purified solid was got to confirm the formation of the intermediate. The NMR of the intermediate obtained is shown in Figure 1.

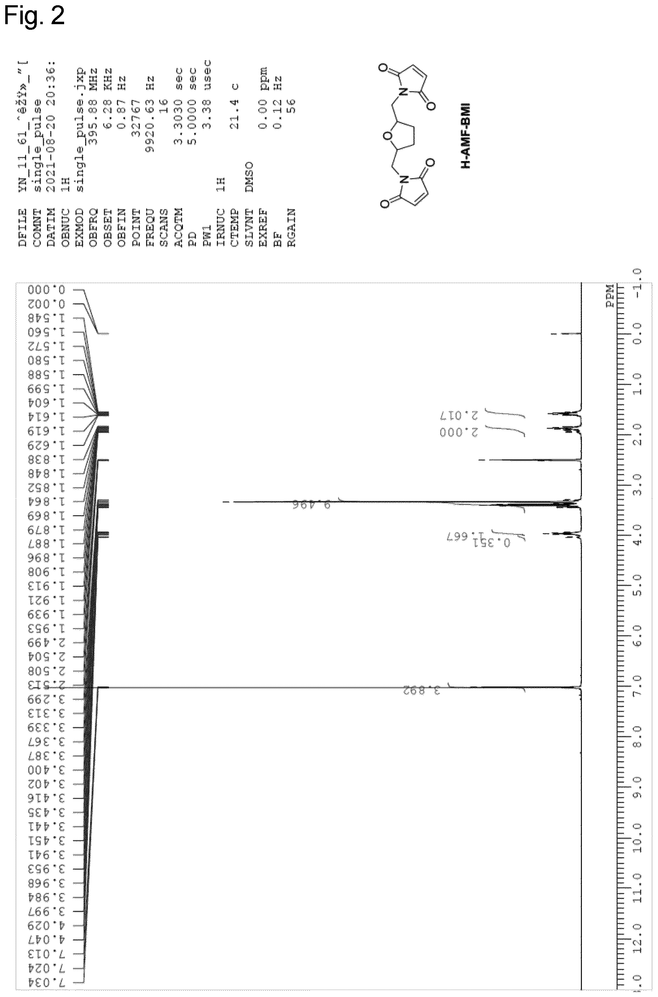

Next, 5.84 g of toluene sulfonic acid hydrate (produced by FUJIFILM Wako Pure Chemical Corporation, molecular weight 190.2) was added to the flask and heated at 140°C for 6.5 hours. After radiational cooling, it was left to stand overnight, then divided, dehydrated with magnesium sulfate, and dried to obtain 18.34 g of a yellow solid (yield 41.1%). NMR of the yellow solid obtained was got to confirm formation of the compound represented by Formula (1) (hereinafter sometimes referred to as H-AMF-BMI). The NMR of the compound obtained is shown in Figure 2.

### <Example 2>

The yellow solid obtained above was heated in a vacuum press machine using a die of 70 mm × 30 mm, made of SUS (Steel Use Stainless), at 5°C/min to 220°C and then held at 250°C for 3 hours while continuously pressing under a condition of 2 MPa to obtain a cured product of 1 mm thickness.

### <Measurement and evaluation methods>

### (1) Glass transition temperature (Tg)

The glass transition temperature was determined as follows: for a sample of 12.7 mm × 30 mm downsized from the cured product obtained, dynamic viscoelasticity was measured using a dynamic viscoelasticity measuring device, in accordance with JIS-K7244-4:1999 (Plastics-Determination of dynamic mechanical properties-Part 4: Tensile vibration-Non resonance method), at a starting temperature of 30°C, a final temperature of 300°C, a heating rate of 5°C/min, a measurement frequency of 1 Hz, and under a nitrogen atmosphere, and the temperature at which the loss tangent (Tan δ) obtained became the maximum value was set as the glass transition temperature.

The dynamic viscoelasticity measuring device, EXSTAR6000 DMS6100 produced by Seiko Instruments Inc. was used.

### (2) Coefficient of thermal expansion (CTE: Coefficient of linear Thermal Expansion)

For the cured product obtained, the coefficient of thermal expansion was measured by the TMA (Thermo-Mechanical Analysis) method specified in JIS C 6481 5.19. Specifically, for a sample of 5 mm × 5 mm downsized from the cured product obtained, a temperature was raised at a heating rate of 10°C/min from 30°C to 340°C using a thermo-mechanical analyzer (produced by TA Instruments) to measure the coefficient of linear thermal expansion (CTE(Z)) (unit: ppm/°C) in the direction perpendicular to the surface from 30°C to 300°C. ppm is the volume ratio. Other details are in accordance with the above JIS C 6481 5.19.

### <Comparative Example 1>

In Example 2, the yellow solid was changed to the same amount of 4,4'-bismaleimidodiphenylmethane (BMI-H, produced by K·I Chemical Industry Inc., LTD.), and others are performed in the same manner.

### <Comparative Example 2>

In Example 2, the yellow solid was changed to the same amount of 1,6'-bismaleimido-(2,2,4-trimethyl)hexane (BMI-TMH, produced by Daiwakasei Industry Co., LTD.), and others are performed in the same manner.

**[Table 1]**

| | Example 2 | Comparative Example 1 | Comparative Example 2 |
|---|---|---|---|
| Bismaleimide | H-AMF-BMI | BMI-H | BMI-TMH |
| Tg | 300°C or more | 146°C | 300°C or more |
| CTE | 60.11 | 54.6 | 116.1 |

As shown in Table 1 above, the cured product of Example was a cured product having a low coefficient of thermal expansion while maintaining excellent heat resistance.

## Claims

1. A compound represented by Formula (1): 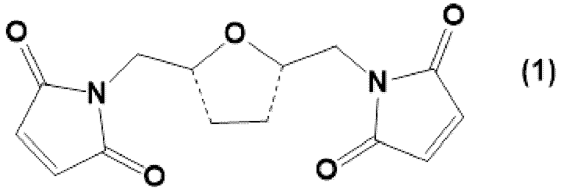 wherein the dotted line portions each independently represent *-CH-CH₂- or *-C=CH-, and * is bonded to the side near the oxygen atom contained in the ring.

2. The compound according to claim 1, wherein the compound represented by Formula (1) is a compound represented by Formula (2):

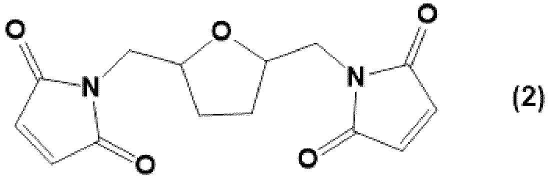

3. A compound represented by Formula (4): 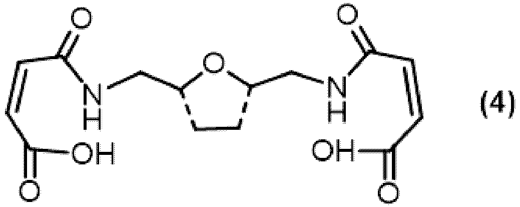 wherein the dotted line portions each independently represent *-CH-CH₂- or *-C=CH-, and * is bonded to the side near the oxygen atom contained in the ring.

4. A composition comprising a compound according to claim 1 or 2.

5. A cured product formed from a composition according to claim 4.

6. A method for producing a compound according to claim 1 or 2, the method comprising using an intermediate represented by Formula (4): 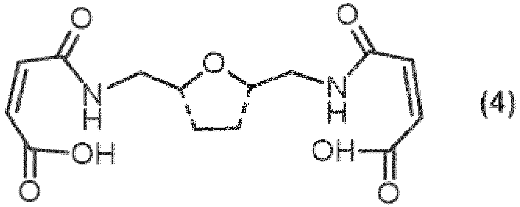 wherein the dotted line portions each independently represent *-CH-CH₂- or *-C=CH-, and * is bonded to the side near the oxygen atom contained in the ring.
